# EUROPEAN PATENT SPECIFICATION

(11) **EP 0 918 548 B1**
(45) Date of publication and mention of the grant of the patent: **20.08.2003**
(21) Application number: 97928372.8
(22) Date of filing: 27.06.1997
(51) Int. Cl.: A61L 15/28, A61L 15/64, A61K 31/717, A61P 17/02

(54) **USE OF OXIDIZED CELLULOSE AND COMPLEXES THEREOF FOR CHRONIC WOUND HEALING**
VERWENDUNG VON OXYDIERTER ZELLULOSE UND IHRER KOMPLEXEN ZUR BEHANDLUNG VON CHRONISCHEN WUNDEN
EMPLOI DE CELLULOSE OXYDEE ET DE COMPLEXES DE LADITE CELLULOSE POUR LA GUERISON DE PLAIES CHRONIQUES

(30) Priority: 28.06.1996 GB 9613682
(43) Date of publication of application: 02.06.1999
(60) Divisional application: 03075991.4
(73) Proprietor: Johnson & Johnson Medical Ltd., Edinburgh EH2 4NH (GB)
(72) Inventor: WATT, Paul William, Steeton, West Yorkshire BD20 6UN (GB); HARVEY, Wilson, Skipton, North Yorkshire BD23 3DW (GB); WISEMAN, David, Dallas, TX 75248 (US); LIGHT, Nicholas, Gargrave, North Yorkshire BD23 3RX (GB); SAFERSTEIN, Lowell, West Orange, NJ 07052 (US); CINI, John, Beacon Falls, CT 06403 (US)
(74) Representative: James, Anthony Christopher W.P.
(86) International application number: GB9701725
(87) International publication number: WO98000180

(56) References cited:
- EP-A- 0 049 469
- EP-A- 0 140 596
- EP-A- 0 177 064
- EP-A- 0 562 862
- DATABASE WPI Section Ch, Week 8948 Derwent Publications Ltd., London, GB; Class A96, AN 89-348621 XP002045447 & CS 8 704 580 A (MARYSKA S) , 13 October 1989

## Description

The present invention relates to the use of oxidized celluloses and complexes thereof with structural proteins such as collagen for chronic wound healing.

Oxidized cellulose is produced by the oxidation of cellulose, for example with dinitrogen tetroxide. This process converts primary alcohol groups on the saccharide residues to carboxylic acid group, forming uronic acid residues within the cellulose chain. The oxidation does not proceed with complete selectivity, and as a result hydroxyl groups on carbons 2 and 3 are occasionally converted to the keto form. These ketone units introduce an alkali labile link, which at pH 7 or higher initiates the decomposition of the polymer via formation of a lactone and sugar ring cleavage. As a result, oxidized cellulose is biodegradable and bioabsorbable under physiological conditions.

The preferred oxidized cellulose for practical applications is oxidized regenerated cellulose (ORC) prepared by oxidation of a regenerated cellulose, such as rayon. It has been known for some time that ORC has haemostatic properties. ORC has been available as a haemostatic product called SURGICEL (Registered Trade Mark of Johnson & Johnson Medical, Inc.) since 1950. This product is produced by the oxidation of a knitted rayon material.

A modification of porosity, density and knit pattern led to the launch of a second ORC fabric product, INTERCEED (Registered Trade Mark - Johnson & Johnson Medical, Inc.) which was shown to reduce the extend of post-surgical adhesions in abdominal surgery.

US-A-2517772 (Doub *et al*.) describes improved haemostatic materials obtained by impregnating ORC fabric with thrombin.

EP-A-0437095 describes a neutralised ORC material prepared by contacting an as-synthesised acidic ORC material with a solution of a basic salt of a weak organic acid, such as sodium acetate. The resulting neutralised product is indicated for haemostasis and adhesion prevention.

Collagen, which is a structural protein of animal origin, is known in various forms for use as a wound dressing material.

GB-A-1515963 describes cross-linked collagen-mucopolysaccharide composite materials for use in medical and surgical applications, blood vessel grafts and all forms of surgical prostheses. The composite material contains at least 0.5% by weight of a mucopolysaccharide irreversibly bound to collagen. The mucopolysaccharide is an animal polysaccharide containing hexosamine residues, such as hyaluronic acid, chondroitin sulphate or heparin sulphate. The composite materials are said to exhibit greater resistance to resorption and better blood compatibility than simple collagen materials.

US-A-4614794 describes complexes formed between collagen and polyanionic plant polysaccharides, such as sodium alginate. The complexes are preferably formed by combining the protein and the polysaccharide at a pH which is no higher than the isoelectric point of the protein. The resulting complexes are said to be suitable for a wide variety of medical and surgical applications, including wound dressings. There is no disclosure of the use of oxidized cellulose instead of the polyanionic plant polysaccharide. The specification also teaches that proteins other than collagen, such as fibrin or elastin may be used in the formation of useful protein/polysaccharide complexes.

GB-A-2280850 describes medicated implants for the treatment of periodontal disease comprising a medicated collagen film reinforced with a layer of bioresorbable polymer, which may be ORC. The collagen matrix may contain dispersed ORC fibers or fragments.

EP-A-0177064 describes the use of a knitted oxidised cellulose fabric as a surgical hemostat.

EP-A-0562862 describes bioabsorbable sponge materials for use as wound implants. The materials comprise a collagen sponge matrix having an oriented substructure therein. The matrix and/or substructures may comprise oxidised regenerated cellulose. There is no disclosure of the use of such materials for the treatment of chronic wounds.

GB-A-1006606 describes haemostatic wound dressings comprising oxidised cellulose crystallite aggregates dispersed in a gel matrix. The use of such haemostatic gels in surgery is disclosed.

The above-described collagen or collagen/polysaccharide wound dressing materials provide important advantages. The materials are of natural, biological origin (albeit sometimes chemically modified), and consequently tend to have low antigenicity. The materials are generally bioabsorbable, which reduces the trauma associated with removal of conventional wound dressing materials from the surface of the wound. Furthermore, some of these materials can have positive therapeutic effects on wound healing. For example, some animal mucopolysaccharides such as hyaluronic acid are thought to exert a chemotactic effect on wound healing cells such as fibroblasts, and thereby promote the growth and development of such cells. Nevertheless, there remains a need for improved wound dressing materials of this general type exhibiting still better control of physical properties and biological absorption rates, still better therapeutic effects on wound healing, and reduced cost.

It is an object of the present invention to provide improved wound dressing materials for mammalian, and especially human, chronic wounds, such as venous ulcers, decubitis ulcers and diabetic ulcers. Such chronic wounds generally exhibit little or no bleeding or adhesion to other body tissues, and accordingly oxidized cellulose would not previously have been indicated for the treatment of such wounds.

It has now been found that oxidized cellulose is an effective wound dressing material for the treatment of chronic wounds.

Accordingly, the present invention provides the use of oxidized cellulose for the preparation of a wound dressing material for the treatment of a chronic wound.

Preferably, the oxidized cellulose is oxidized regenerated cellulose (ORC). Preferably, the oxidized cellulose is in the form of a woven, non-woven or knitted fibrous body that is insoluble in wound fluids. For example, the oxidized cellulose may be in the form of one of the SURGICEL (RTM) or INTERCEED (RTM) fabrics known in the art. In other preferred embodiments, the oxidized cellulose is in the form of fibers, such as milled fibers, or powder, preferably dispersed in a suitable topical medicament vehicle.

Preferably, the oxidised cellulose has an average molecular weight greater than 50,000. Such oxidised cellulose is substantially insoluble in wound fluids, but will undergo very gradual breakdown into bioresorbable fragments at physiological pH.

Preferably, the oxidized cellulose is not neutralized. However, the present invention encompasses the use of partially or completely neutralised materials as described in EP-A-0437095 for the preparation of medicaments for the treatment of chronic wounds as hereinbefore defined.

Preferably, the oxidized cellulose is complexed with a structural protein in the said wound dressing material.

Preferably, the protein (where present) and oxidized cellulose together make up at least 75% by weight of the wound dressing material, more preferably at least 90% by weight of the material. Other components of the material may include 0-25% by weight of one or more other biocompatible polysaccharides, for example alginates such as sodium alginate or calcium alginate, starch derivatives such as sodium starch glycolate, cellulose derivatives such as methyl cellulose or carboxymethyl cellulose, or glycosaminoglycans such as hyaluronic acid or its salts, chondroitin sulfate or heparan sulfate. The material may also comprise up to 20% by weight, preferably up to 10% by weight of water. The material may also contain 0-40% by weight, preferably 0-25% by weight of a plasticiser, preferably a polyhydric alcohol such as glycerol. The material may also comprise 0-10% by weight, preferably 0-5% by weight of one or more therapeutic wound healing agents, such as non-steroidal anti-inflammatory drugs (eg. acetaminophen), steroids, antibiotics (eg. penicillins or streptomycins), antiseptics (eg. silver sulfadiazine or chlorhexidine), or growth factors (eg. fibroblast growth factor or platelet derived growth factor).

Preferably, the weight ratio of protein to oxidized cellulose is from 1:99 to 99.99:1. More preferably, the weight ratio is in the range 1:10 to 99.9:1, still more preferably it is in the range 2:1 to 95:1.

The material according to the present invention may be in any convenient form, such as a powder, microspheres, flakes, a mat or a film. However, preferably, the material according to the present invention is in the form either of a semisolid or gel ointment for topical application, or of a freeze-dried or solvent-dried sponge.

Preferably, the wound dressing medicament is a sterile packaged semisolid or gel ointment for topical application to a chronic wound, wherein said ointment comprises from 0.05% to 50% w/v of oxidized cellulose.

Preferably, the ointment comprises from 0.1% to 20% w/v, more preferably 1% to 10% w/v, of the oxidized cellulose in a pharmaceutically acceptable carrier. Suitable carriers include: Hydrogels containing cellulose derivatives, including hydroxyethyl cellulose, hydroxymethyl cellulose, carboxymethyl cellulose, hydroxypropylmethyl cellulose and mixtures thereof; and hydrogels containing polyacrylic acid (Carbopols). Suitable carriers also include creams/ointments used for topical pharmaceutical preparations, e.g. creams based on cetomacrogol emulsifying ointment. The above carriers may include alginate (as a thickener or stimulant), preservatives such as benzyl alcohol, buffers to control pH such as disodium hydrogen phosphate/sodium dihydrogen phosphate, agents to adjust osmolarity such as sodium chloride, and stabilisers such as EDTA.

Preferably, the oxidized cellulose in the ointment is complexed to a protein, as described herein.

Alternatively, the wound dressing is a freeze-dried or solvent-dried bioabsorbable sponge for application to a chronic wound, the sponge comprising from 0.1% to 50% w/w of oxidized cellulose and from 50% to 99.9% w/w of one or more structural proteins. Preferably, the average pore size of the sponge is in the region of 10-500 µm, more preferably about 100-300µm.

Suitable proteins for complexation to oxidized cellulose in this invention include the structural proteins such as fibronectin, fibrin, laminin, elastin and collagen. Preferably the protein comprises collagen, and more preferably it consists essentially of collagen. The collagen may be collagen obtained from any natural source, including microbiological sources, but is preferably collagen obtained from bovine corium that has been rendered largely free of non-collagenous components, for example fat, non-collagenous proteins, polysaccharides and other carbohydrates as described in US Patents Nos. 4614794 and 4320201, the entire contents of which are hereby incorporated by reference. The collagen may be Type I, II or III collagen, or may also be chemically modified collagen, for example an atelocollagen obtained by removing the immunogenic telopeptides from natural collagen. The collagen may also comprise solubilised collagen or soluble collagen fragments having molecular weights in the range 5,000-100,000, preferably 5,000-50,000 obtained by pepsin treatment of natural collagen in known fashion.

Preferably, the oxidized cellulose comprises oxidized regenerated cellulose (ORC). The oxidized regenerated cellulose (ORC) can be obtained by the process described in US Patent No. 3122479, the entire content of which is incorporated herein by reference. This material offers numerous advantages including the features that it is biocompatible, biodegradable, non-immunogenic and readily commercially available. ORC is available with varying degrees of oxidation and hence rates of degradation. The ORC may be used in the form of insoluble fibers, including woven, non-woven and knitted fabrics. In other preferred embodiments, the ORC is in the form of water-soluble low molecular weight fragments obtained by alkali hydrolysis of ORC.

The ready availability of both collagen and ORC having a range of controllable properties means that the properties of the materials used in the present invention can be controlled to an exceptional degree. In particular, the rate of biological absorption, porosity and density of the materials can be controlled.

It has also been found, surprisingly, that the protein/oxidized cellulose complexes used in the preferred aspect of the present invention have an excellent ability to bind to growth factors, in particular, platelet derived growth factor.
In the use according to the present invention the medicament may be an active wound dressing material, wherein the preparation comprises the steps of:
(i) contacting a material comprising oxidized cellulose or a complex of oxidized cellulose with a structural protein with a biological medium containing cell growth factors to bind the cell growth factors to the material; and
(ii) washing and drying the material having the cell growth factors bound thereto to form said active wound dressing material.
In these embodiments the cell growth factors preferably comprise platelet derived growth factor.

The use according to the present invention provides medicaments for use in methods of treatment of a chronic wound in a mammal, such as a decubitis ulcer, a venous ulcer or a diabetic ulcer. The method comprises applying a dressing comprising oxidized cellulose or a complex thereof with a structural protein as herinbefore defined to the wound.

Preferably, the oxidized cellulose is applied to the chronic wound for a period of at least 1 hour, more preferably at least 6 hours, and most preferably at least 12 hours. The oxidized cellulose treatment may be extended for several days or weeks, with oxidized cellulose dressing changes as appropriate, if necessary for chronic wounds. This contrasts with haemostatic applications of ORC, which typically last only a few seconds or minutes.

Without wishing to the bound by any theory, it is thought that the oxidized cellulose and complexes thereof promote chronic wound healing in at least some of the following ways. Firstly, the oxidized cellulose binds to growth factors such as PDGF, EGF and FGF to retain these growth factors at the wound site. Otherwise, such growth factors tend to be carried away from the wound site along with the wound exudate. The gradual breakdown of oxidized cellulose at physiological pH results in gradual release of the growth factors back into the wound. A second reason is that oxidized cellulose is fully bioresorbable and physiologically acceptable. A third reason may be that the oligosaccharide fragments produced by the breakdown of oxidized cellulose *in vivo* themselves promote chronic wound healing.

Preferably, the chronic wound is selected from the group consisting of venous ulcers, decubitis ulcers and diabetic ulcers. Preferably, the chronic wound is substantially or completely non-bleeding. The term "chronic wound" does not encompass a periodontal disorder or disease.

The protein/oxidized cellulose complexes used in the present invention can be made by a process comprising the steps of: providing an aqueous dispersion of a protein; immersing or dispersing oxidized cellulose in the aqueous dispersion; following by removing water from the aqueous dispersion to leave a material comprising protein complexed with oxidized regenerated cellulose.

The optional, additional components in the materials according to the present invention are preferably included in the aqueous dispersion prior to removal of water from the aqueous dispersion.

Preferably, the pH of the dispersion is adjusted to pH 3-4.5. This pH range is less than the isoelectric pH of collagen.

The water can be removed from the aqueous dispersion by evaporation, for example by evaporation from the dispersion in a tray to leave a film of material. However, preferably, the water is removed by freeze-drying (lyophilizing) or solvent-drying to produce the material in the form of a sponge. Preferably, the aqueous dispersion contains 5-30mg/ml of collagen. Preferably, a collagen-based sponge is formed by lyophilisation substantially as described in US-A-2157224.

Preferably, the process further comprises treating the protein and polysaccharide in the dispersion, or in the dried material, with a cross-linking agent such as carbodiimide, hexamethylene diisocyanate (HMDI) or glutaraldehyde.
Alternatively, cross-linking may be carried out dehydrothermally. The method of cross-linking can markedly affect the final product. For example, HMDI cross-links the primary amino groups on the protein within the complex, whereas carbodiimide cross-links carbohydrate on the ORC to primary amino groups on the protein.

The oxidized cellulose may be added to the aqueous dispersion of protein in the form of a suspension or solution of the oxidized cellulose, preferably at a comparable pH to the collagen suspension, following by mixing by stirring or homogenisation. Alternatively, dry fibers or fabric of oxidized cellulose may be immersed in the aqueous dispersion of collagen.

Specific embodiments of the present invention will now be described further, by way of example, with reference to the accompanying drawings, in which:
Figure 1 shows a graph of fibroblast cell growth (in arbitrary units) on serum treated films consisting of (a) pepsin solubilised collagen, (b) pepsin solubilised collagen complexed with ORC fragments having average molecular weight 8,000, (c) pepsin solubilised collagen complexed with ORC fragments having average molecular weight about 20,000, and (d) pepsin solubilised collagen complexed with heparan sulphate (for comparison);
Figure 2 shows a graph of percentage binding of platelet derived growth factor (PDGF) to the following materials: (a) plastics, (b) collagen sponge, (c) collagen/solubilised ORC sponge, (d) INTERCEED (RTM) ORC, and the ease of removal of this growth factor from the materials; and
Figure 3 shows a graph of relative amounts of MMP binding measured for a collagen sponge and SURGICEL (RTM) ORC fabric (comparative measurements) and for sponges of collagen complexed with 10 wt.%, 20wt.% and 30 wt.% of fibrous ORC.

### Example 1: Preparation of a collagen/fibrous ORC sponge

Lyophilised collagen, prepared as described in US Patent No. 4614794 of 4320201, is re-suspended in cold 0.05M acetic acid at a concentration of 10mg/ml. Milled ORC powder (milled Surgicel® cloth) is added to the suspension in a ratio of 1:3 ORC:collagen and homogenised using a Waring Blendor on low speed for 3 x 30s. The complex suspension is degassed in a vacuum oven for 10 min. and then poured to a depth of 3mm and blast frozen. The frozen suspension is then either freeze-dried and dehydrothermally cross-lined using a programmable Edwards freeze-drier with a temperature ramping facility, or dried using a solvent drying process as described in US-A-2157524.

### Example 2: Preparation of a collagen/ORC oligosaccharide sponge

Soluble collagen is prepared by the published method of E.J. Miller and R.K. Rhodes "Preparation and Characterisation of the Different Types of Collagen", Methods Enzymol Vol. 82, pages 33-64 (1982). Soluble oligosaccharides of ORC are prepared as described in a copending Patent Application filed on the same date as this application and commonly assigned herewith. Briefly the ORC oligosaccharides are prepared by treating commercially available ORC with 6M sodium hydroxide solution at 37°C for 45 minutes, followed by neutralization and dialysis to remove fragments and impurities having molecular weight below 1000. The resulting soluble oligosaccharides of ORC are slowly added while stirring to soluble collagen (.075mg/ml) (both in cold 0.05M acetic acid) until no further precipitation of the complex occurs. The complex precipitate is isolated by centrifugation, washed in phosphate buffer at pH 7.2, and re-suspended, by homogenisation, at 30% w/v in the same buffer. The suspension is poured to a depth of 3mm, blast frozen at - 30°C and freeze-dried.

### Example 3: Preparation of a collagen/ORC film

A collagen/ORC film for application to a wound is made by the methods described in either of the first two examples, except that the suspensions are not precipitated but air-dried rather than frozen and freeze-dried. To prepare flexible films a small amount of glycerol may be added to the suspensions.

### Example 4: Preparation of collagen/ORC sponge using a pre-gelling process of the ORC

ORC fabric (SURGICEL) is suspended (4% w/v) in dilute alkali (NaHCO₃) at pH 8.0 for a time that is sufficient to convert the fabric to a gelatinous mass. Collagen slurry is added at the same pH to give a final solids content of both Surgicel® and collagen of 1% w/v. The slurry is stirred and the pH adjusted to pH 3.0-4.0 using acetic acid. The final slurry is moulded, frozen and freeze-dried under vacuum.

### Example 5

A wound dressing suitable for application to a venous ulcer, decubitis ulcer or diabetic ulcer is prepared by cutting 5cm x 5cm² of INTERCEED (RTM) fabric obtained from Johnson & Johnson Medical, Inc.

### Example 6

A wound treatment gel for topical application to a wound is prepared as follows.

SURGICEL (RTM) fabric is milled through a 1mm screen and the resulting fibers are dispersed at 3.0% w/w concentration in a 3.0% w/w carboxymethyl cellulose (CMC) aqueous gel. The gel is packaged in a metallized polymer pouch and sterilized by γ-irradiation.

### Example 7

A neutralized ORC wound dressing suitable for application to a venous ulcer, decubitis ulcer or diabetic ulcer was prepared as described in EP-A-0437095. Briefly, a quantity of 71.9 grams of oxidized regenerated cellulose (ORC) fabric with a carboxylic acid content of 15.2% available as INTERCEED (RTM) from Johnson & Johnson Medical, Inc., was wrapped around a perforated core and placed in a reactor equipped with a circulating pump. The reactor was filled with a mixture of 3 liters of water 2 liters of methyl alcohol. A Brookfield EX-100 constant temperature bath with built-in pump for external circulation of solutions was turned on to pump the solvent through the centre of the perforated core and through the piles of cloth wrapped around the core. The solvent flowed through the exit line and recirculated again through the pump and back to the core.

A stoichiometric amount of sodium acetate trihydrate equal to the number of moles of carboxylic acid on the cloth was added to the solution, i.e. 71.9 x 15.2% = 10.92 grams of carboxylic acid; equivalent to 33.05 grams of sodium acetate trihydrate.

The 33.05 grams of sodium acetate trihydrate were added to the aqueous alcohol solution and circulated by pump around and through the fabric for 30 minutes. After 30 minutes the pH of the circulating solution reached a constant value (pH 4.6). The fabric was then removed from the reactor and placed in 600 ml of methanol for 10 minutes. the methanol was removed and replaced with another 600 ml of fresh methanol. After the second wash, the cloth was hung up and air dried. A 5cm x 5cm piece of the cloth was cut for use as a wound dressing in accordance with the present invention.

The advantageous properties of the materials according to the present invention prepared as above were determined as follows:

### Procedure 1: Promotion of fibroblast cell growth

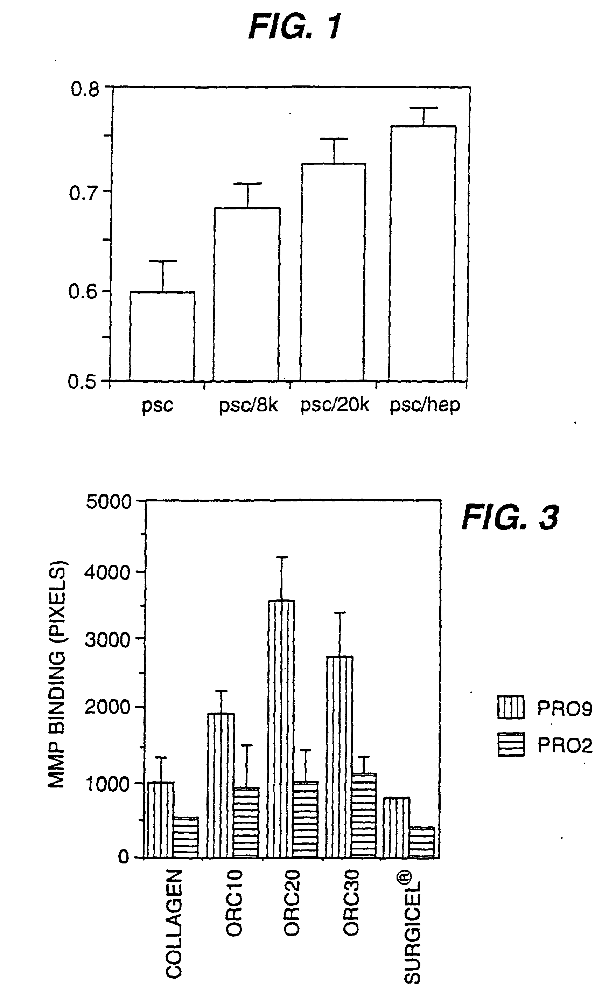

A collagen/alkali-soluble ORC complex film was prepared in a Petri dish as described in Examples 2/3, and serum poured over the film and incubated at 37°C overnight. The serum was removed and the effects on fibroblast cell growth measured (Fig. 1). Cell growth was observed for a pepsin solubilised collagen (PSC) film (control), PSC plus ORC oligosaccharides having average molecular weights of about 8,000 and about 20,000 prepared as described above, and PSC plus heparin was included as a positive control. The results show that the collagen/ORC fragments film appears to bind factors from the serum which stimulate cell growth.

### Procedure 2: Binding of platelet derived growth factor

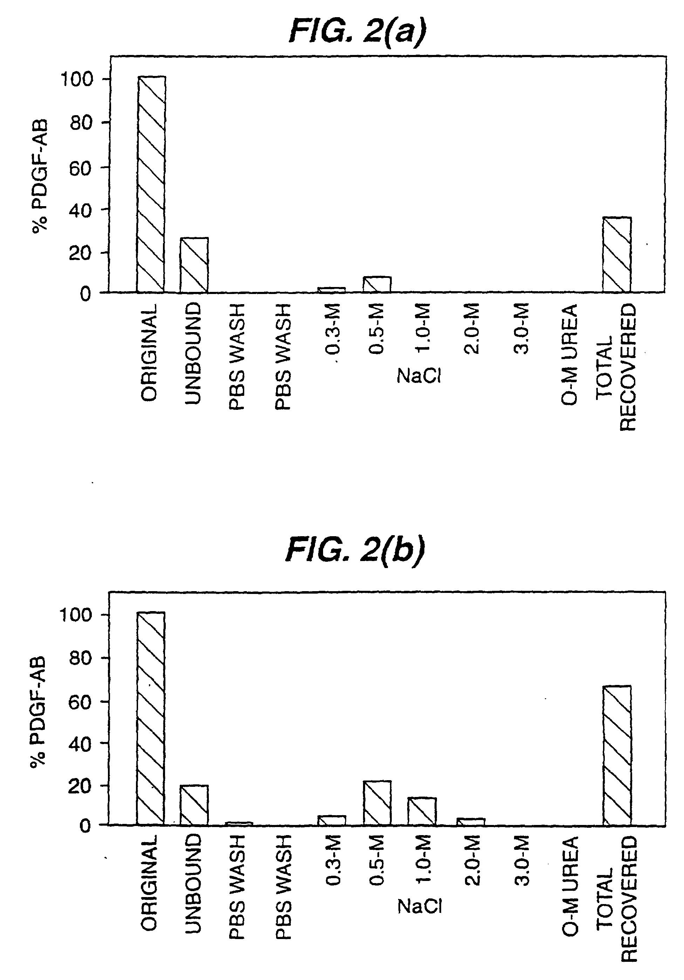

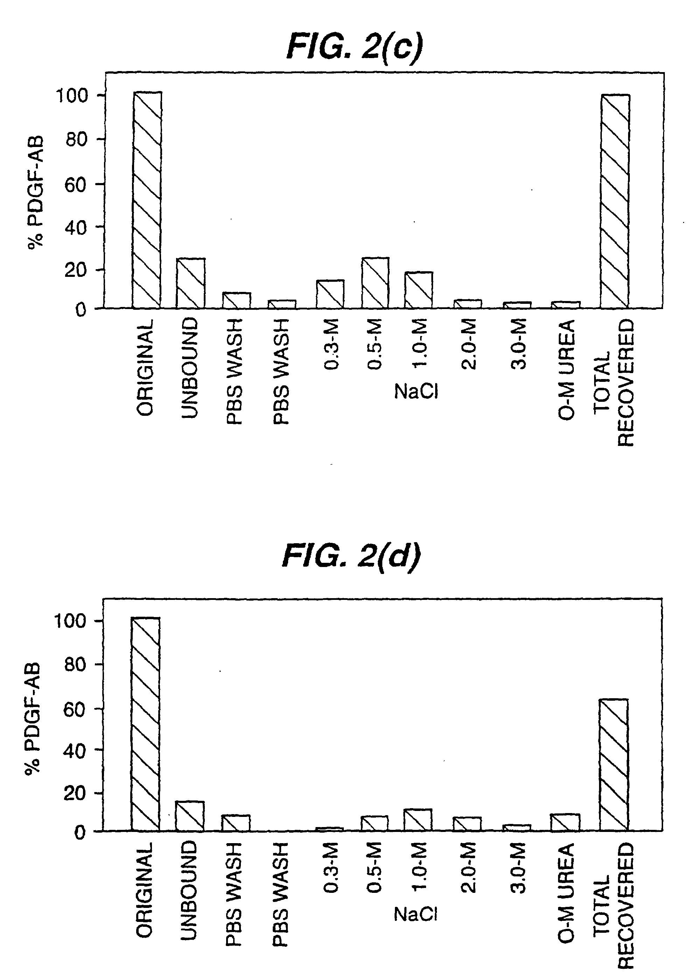

PDGF binding studies were carried out as follows: Small sections of test material (approximately 1cm² squares of INTERCEED (RTM) ORC fabric, and approximately 1cm x 0.5cm x 0.4cm sections of collagen sponge) were weighed and soaked in 100mM sodium phosphate dibasic buffer containing 150mM sodium chloride (total volume 1ml) for at least one hour at room temperature. Samples were then incubated with 2% bovine serum albumin (BSA) in phosphate buffered saline (PBS) for 2 hours at room temperature. 22ng of PDGF was then added to each sample in 250µl of PBS containing 2% BSA, and samples were then incubated for a further hour at 37°C. Each sample was then washed three times with 250µl PBS, followed by increasing concentrations of sodium chloride. Finally, each sample was washed with 4.0M urea. PDGF ELISA analyses of the original PDGF preparation and the various washings were carried out. The data shown in Fig. 2 indicate that the growth factor can be totally recovered from the composite of collagen and ORC while the individual components appear not to release all the growth factor. The binding characteristics are also uniquely different for the collagen/ORC complex compared with the individual components. These observations indicate that the complex has unique binding of PDGF which may be utilised appropriately for both exogenous binding and endogenous binding and release of growth factor.

### Procedure 3: Matrix Metalloproteinase Binding

The effect of complexation between collagen and ORC on matrix metalloproteinase (MMP) binding was assessed as follows.

Collagen/fibrous ORC sponges containing 0%, 20% and 30% by weight of fibrous ORC were prepared by the procedure described in Example 1. An ORC-free collagen sponge was prepared for comparison purposes. A sample of SURGICEL (RTM) ORC fabric was also prepared for comparison.

Briefly 50mg of each material was placed in a 15ml plastic beaker containing 2.5ml of an acute wound fluid diluted to 1:50 in a proteolysis buffer (50mM tris/HCl pH7.8, 50mM CaCl₂, 0.5M NaCl) and incubated at 37°C on a shaking water bath for 3 hours. Acute wound fluid contains various proteinases, including matrix metalloproteinases and many of these enzymes will preferentially bind to various dressing materials. The excess fluid absorbed by each material was mechanically expressed using a metal spatula and discarded. The remaining dressings were placed into pre-packed 2ml syringes (each syringe contained 0.5ml volume of 2.5mm glass beads). 4ml of proteolysis buffer was forced through the syringe in 1ml aliquots which were discarded. At this washing stage all of the unbound proteinases and proteinases which were only weakly bound to the dressing material had been removed from the dressing leaving the more tightly bound forms. The buffer rinsed dressings were then removed to another 15ml plastic beaker. 1ml of non-denaturing sample buffer (6.3ml 0.05M tris/HCl.c pH6.8, 2.5ml glycerol, 0.5g SDS, 16.2ml water and bromophenol blue) was added to each sample which were placed on an orbital shaker at setting six for 2 hours. The sample buffer detaches the tightly bound proteinases from the materials which are then present in the sample buffer itself. After this time 20 microliters of sample buffer was taken from each container and subjected to gelatin substrate SDS-polyacrylamide gel electrophoresis (zymography), as described by Heussen C. and Dowdle E.B., Anal. Biochem. 102:196-202 (1980).

The areas of the individual zones of clearance on the gels, which are due to proteinase activity, were accurately measured by the Optilab® system. This was achieved by repeating each binding experiment (n=3) and analyzing the results statistically by the Students T test, where P ≤ 0.05. Analysis was against controls of pure collagen.

The results, shown in Figure 3, demonstrate a surprising synergistic improvement in MMP binding for the complexes of collagen with ORC. Data are presented for the proenzyme forms (PR02 and PR09) of matrix metalloproteinase 2 (Gelatinase A) and matrix metalloproteinase 9 (Gelatinase B). Without wishing to be bound by an theory, it is thought that the improvement may be related to neutralization of opposing electrostatic charges on the collagen and the ORC by complexation.

### Procedure 4

The binding of purified growth factors to pure ORC and neutralised ORC cloth was investigated as follows. Samples of 200 micrograms of PDGF, EGF and FGF were added to 1cm x 1cm squares of INTERCEED (RTM) and neutralised Interceed (from Example 2) and the amount of unbound growth factor was measured by HPLC. The study investigated both the effect of ionic strength and the effect of carboxylation upon the growth factor binding. The ORC materials were incubated with growth factor for 1 hour at 37°C, then washed with 2 x 1 ml of 0.05 or 0.03M NaCl for 5 minutes. The wash solutions were collected, pooled and assayed for growth factor by HPLC. The results are shown in Table 1.

**TABLE 1**

| Interceed™ | pH | % COOH | % EGF Bound | | % FGF Bound | | % PDGF Bound | |
|---|---|---|---|---|---|---|---|---|
| | | | Low Salt | High Salt | Low Salt | High Salt | Low Salt | High Salt |
| Interteed™ | 2.0 | 15% | 97 | 87 | 100 | 100 | 97 | 95 |
| nTC7 | 5.4 | 15% | 0 | 0 | 0 | 98 | 84 | 99 |
| | | | | | | | | |
| Interceed™ | 2.0 | 6% | 97 | 33 | 91 | 89 | 98 | 64 |
| nTC7 | 5.4 | 6%0 | 0 | 0 | 100 | 84 | 100 | 84 |

The results show that between 85 and 100% PDGF, FGF and EGF bind to INTERCEED. However, only FGF and EGF bind to neutralised INTERCEED. It can be postulated that EGF does not bind because of the difference in isoelectric points of the growth factors (PDGF = 9.6, FGF = 10 and EGF = 4.8).

The above examples are intended for the purpose of illustration only. Many other embodiments falling within the scope of the accompanying claims will be apparent to the skilled reader.

## Claims

1. Use of oxidized cellulose for the preparation of a medicament for the treatment of a chronic wound.

2. Use according to Claim 1, wherein the oxidized cellulose is in the form of a woven, nonwoven or knitted fibrous body, that is insoluble in wound fluids.

3. Use according to any preceding claim, wherein said oxidized cellulose is in the form of dispersed fibers or powders.

4. Use according to claim 3, wherein said fibers or powder are dispersed in a semi-solid vehicle for topical application.

5. Use according to any preceding claim, wherein the oxidized cellulose has average molecular weight greater than 50,000.

6. Use according to any preceding claim, wherein the oxidized cellulose comprises oxidized regenerated cellulose (ORC).

7. Use according to any preceding claim, wherein the oxidized cellulose is complexed with a structural protein.

8. Use according to claim 7, wherein the oxidized cellulose and structural protein (where present) together make up at least 75% by weight of the material.

9. Use according to claim 8, wherein the oxidized cellulose and structural protein (where present) together make up at least 90% by weight of the material.

10. Use according to claim 7, 8 or 9, wherein the material is a freeze-dried or solvent-dried sponge.

11. Use according to claim 7, 8 or 9, wherein the material is a solid film.

12. Use according to any of claims 7 to 11, wherein the weight ratio of protein to oxidized cellulose is from 1:99 to 99.99:1.

13. Use according to claim 12, wherein the weight ratio of protein to oxidized cellulose is in the range 1:10 to 99.9:1.

14. Use according to any of claims 7 to 13, wherein the protein comprises collagen, fibronectin, fibrin, laminin or elastin.

15. Use according to claim 14, wherein the protein consists essentially of collagen.

16. Use according to claim 14 or 15, wherein the protein comprises partially hydrolyzed, soluble collagen having molecular weights in the range 5,000-100,000.

17. Use according to claim 14 or 15, wherein the collagen is fibrous, substantially insoluble collagen.

18. Use according to any preceding claim, wherein the oxidized cellulose comprises water-soluble oxidized cellulose fragments having molecular weights in the range 5,000-50,000.

19. Use according to any preceding claim wherein said chronic wound is selected from the group consisting of venous ulcers, decubitis ulcers and diabetic ulcers.

20. Use according to claim 1, wherein the medicament is an active wound dressing material and said preparation comprises the steps of:
(i) contacting a material comprising oxidized cellulose or a complex of oxidized cellulose with a structural protein with a biological medium containing cell growth factors to bind the cell growth factors to the material; and
(ii) washing and drying the material having the cell growth factors bound thereto to form said active wound dressing material.

21. Use according to claim 20, wherein the cell growth factors comprise platelet derived growth factor.

22. Use according to claim 1, wherein said medicament is sterile packaged semisolid or gel ointment for topical application to a chronic wound, wherein said ointment comprises from 0.05% to 50% w/v of said oxidized cellulose.

23. Use according of claim 1, wherein said medicament is bioabsorbable sponge for application to a chronic wound, said sponge comprising from 0.1% to 50% w/w of said oxidized cellulose and from 50% to 99.9% of one or more structural proteins.

## Patentansprüche

1. Verwendung von oxidierter Zellulose zur Präparation eines Medikaments zur Behandlung einer chronischen Wunde.

2. Verwendung nach Anspruch 1, wobei die oxidierte Zellulose in Form eines gewebten, nicht gewobenen oder gewirkten fasrigen Körpers vorliegt, der in Wundflüssigkeit unlöslich ist.

3. Verwendung nach einem der voranstehenden Ansprüche, wobei die oxidierte Zellulose in Form von dispergierten Fasern oder Pulvern vorliegt.

4. Verwendung nach Anspruch 3, wobei die Fasern oder Pulver in einem semi-festen Vehikel zur topischen Applikation dispergiert vorliegen.

5. Verwendung nach einem der voranstehenden Ansprüche, wobei die oxidierte Zellulose ein durchschnittliches Molekulargewicht von mehr als 50.000 aufweist.

6. Verwendung nach einem der voranstehenden Ansprüche, wobei die oxidierte Zellulose oxidierte regenerierte Zellulose (ORC) umfaßt.

7. Verwendung nach einem der voranstehenden Ansprüche, wobei die oxidierte Zellulose mit einem strukturellen Protein komplexiert vorliegt.

8. Verwendung nach Anspruch 7, wobei die oxidierte Zellulose und das strukturelle Protein (wenn vorhanden) zusammen mindestens 75 Gew.-% des Materials ausmachen.

9. Verwendung nach Anspruch, wobei die oxidierte Zellulose und das strukturelle Protein (wenn vorhanden) zusammen mindestens 90 Gew.-% des Materials ausmachen.

10. Verwendung nach Anspruch 7, 8 oder 9, wobei das Material ein gefriergetrockneter oder Lösungsmittel-getrockneter Schwamm ist.

11. Verwendung nach Anspruch 7, 8 oder 9, wobei das Material ein fester Film ist.

12. Verwendung nach einem der Ansprüche7 bis 11, wobei das Gewichtsverhältnis von Protein zu oxidierter Zellulose von 1:99 bis 99,99:1 ist.

13. Verwendung nach Anspruch 12, wobei das Gewichtsverhältnis von Protein zu oxidierter Zellulose in Bereiche von 1:10 bis 99,9:1 ist.

14. Verwendung nach einem der Ansprüche 7 bis 13, wobei das Protein Collagen, Fibronectin, Fibrin, Laminin oder Elastin umfaßt.

15. Verwendung nach Anspruch 14, wobei Protein im wesentlichen aus Collagen besteht.

16. Verwendung nach Anspruch 14 oder 15, wobei das Protein partiell hydrolisiertes, lösliches Collagen umfaßt, das Molekulargewichte im Bereich von 5.000-100.000 aufweist.

17. Verwendung nach Anspruch 14 oder 15, wobei das Collagen fasriges, im wesentlichen unlösliches Collagen ist.

18. Verwendung nach einem der voranstehenden Ansprüche, wobei die oxidierte Zellulose wasserlösliche oxidierte Zellulosefragmente umfaßt, die Molekulargewichte im Bereich von 5.000-50.000 aufweist.

19. Verwendung nach einem der voranstehenden Ansprüche, wobei die chronische Wunde ausgewählt ist aus der Gruppe, bestehend aus venösen Ulzern, Decubitisulzem und diabetischen Ulzern.

20. Verwendung nach Anspruch 1, wobei das Medikament ein aktives Wundverbandmaterial ist und die Herstellung die Schritte umfaßt von:
(i) in Kontakt Bringen eines Materials, das oxidierte Zellulose oder einen Komplex von oxidierter Zellulose mit einem strukturellen Protein umfaßt, mit einem biologischen Medium, das Zellwachstumsfaktoren enthält, um die Zellwachstumsfaktoren an das Material zu binden; und
(ii) Waschen und Trocknen des Materials, das die daran gebundenen Zellwachstumsfaktoren aufweist, um das aktive Wundverbandmaterial zu bilden.

21. Verwendung nach Anspruch 20, wobei die Zellwachstumsfaktoren Plättchen-abgeleiteten Wachstumsfaktor umfassen.

22. Verwendung nach Anspruch 1, wobei das Medikament eine steril verpackte semifeste- oder Gel-Einreibung zur topischen Applikation auf eine chronische Wunde ist, wobei die Einreibung von 0,05% bis 50% w/v der oxidierten Zellulose umfaßt.

23. Verwendung nach Anspruch 1, wobei das Medikament ein bioabsorptionsfähiger Schwamm zur Aufbringung auf eine chronische Wunde ist, wobei der Schwamm von 0,1% bis 50% w/w der oxidierten Zellulose umfaßt und 50% bis 99,9% von einem oder mehreren strukturellen Proteinen.

## Revendications

1. Utilisation de cellulose oxydée pour la préparation d'un médicament pour le traitement d'une plaie chronique.

2. Utilisation selon la revendication 1, dans laquelle la cellulose oxydée est sous forme d'un corps fibreux tissé, non tissé ou tricoté, qui est insoluble dans les fluides de la plaie.

3. Utilisation selon l'une quelconque des revendications précédentes, dans laquelle ladite cellulose oxydée est sous forme de fibres ou de poudres dispersées.

4. Utilisation selon la revendication 3, dans laquelle lesdites fibres ou poudre sont dispersées dans un vecteur semi-solide pour une application topique.

5. Utilisation selon l'une quelconque des revendications précédentes, dans laquelle la cellulose oxydée a un poids moléculaire moyen supérieur à 50 000.

6. Utilisation selon l'une quelconque des revendications précédentes, dans laquelle la cellulose oxydée comprend de la cellulose oxydée régénérée (ORC).

7. Utilisation selon l'une quelconque des revendications précédentes, dans laquelle la cellulose oxydée est complexée avec une protéine structurale.

8. Utilisation selon la revendication 7, dans laquelle la cellulose oxydée et une protéine structurale (si elle est présente) constituent ensembles au moins 75 % du poids du matériau.

9. Utilisation selon la revendication 8, dans laquelle la cellulose oxydée et la protéine structurale (si elle est présente) constituent ensemble au moins 90 % du poids du matériau.

10. Utilisation selon les revendications 7, 8 ou 9, dans laquelle le matériau est une éponge lyophilisée ou séchée sur solvant.

11. Utilisation selon les revendications 7, 8 ou 9, dans laquelle le matériau est un film solide.

12. Utilisation selon l'une quelconque des revendications 7 à 11, dans laquelle le rapport en poids de protéine par rapport à la cellulose oxydée est compris entre 1 : 99 et 99,99 : 1.

13. Utilisation selon la revendication 12, dans laquelle le rapport en poids de protéine par rapport à la cellulose oxydée, se situe dans le domaine compris entre 1 : 10 et 99,9 : 1.

14. Utilisation selon l'une quelconque des revendications 7 à 13, dans laquelle la protéine comprend le collagène, la fibronectine, la fibrine, la laminine ou l'élastine.

15. Utilisation selon la revendication 14, dans laquelle la protéine est constituée essentiellement de collagène.

16. Utilisation selon les revendications 14 ou 15, dans laquelle la protéine comprend du collagène soluble, partiellement hydrolysé, ayant des poids moléculaires se situant dans le domaine compris entre 5000 et 100 000.

17. Utilisation selon les revendications 14 ou 15, dans laquelle le collagène est en collagène fibreux, substantiellement insoluble.

18. Utilisation selon l'une quelconque des revendications précédentes, dans laquelle la cellulose oxydée comprend des fragments de cellulose oxydée soluble dans l'eau, ayant des poids moléculaires se situant dans le domaine compris entre 5000 et 50 000.

19. Utilisation selon l'une quelconque des revendications précédentes, dans laquelle ladite plaie chronique est choisie dans le groupe constitué par des ulcères veineux, des escarres de décubitus et des ulcères diabétiques.

20. Utilisation selon la revendication 1, dans laquelle le médicament est un matériau de pansement actif de plaie, et ladite préparation comprend les étapes consistant à :
(i) mettre en contact un matériau comprenant de la cellulose oxydée ou un complexe de cellulose oxydée avec une protéine structurale, avec un milieu biologique contenant des facteurs de croissance cellulaire pour fixer les facteurs de croissance cellulaire au matériau ; et
(ii) laver et sécher le matériau auquel les facteurs de croissance cellulaire sont fixés, pour former ledit matériau de pansement actif de plaie.

21. Utilisation selon la revendication 20, dans laquelle les facteurs de croissance cellulaire comprennent le facteur de croissance dérivé des plaquettes.

22. Utilisation selon la revendication 1, dans laquelle ledit médicament est un onguent semi-solide ou en gel, emballé stérilement, pour une application topique sur une plaie chronique, dans laquelle ledit onguent comprend de 0,05 % à 50 % poids/volume de ladite cellulose oxydée.

23. Utilisation selon la revendication 1, dans laquelle ledit médicament est une éponge bioabsorbable pour une application à une plaie chronique, ladite éponge comprenant de 0,1 % à 50 % poids/volume de ladite cellulose oxydée, et de 50 % à 99,9 % de d'une ou plusieurs protéines structurales.
